# EUROPEAN PATENT APPLICATION

(11) **EP 3 991 684 A2**
(43) Date of publication of application: **04.05.2022**
(21) Application number: 21206031.3
(22) Date of filing: 02.11.2021
(51) Int. Cl.: A61B 34/20, G06T 7/00

(54) **IDENTIFICATION AND VISUALIZATION OF NON-TRACKED OBJECTS IN MEDICAL IMAGES**

(30) Priority: 03.11.2020 US 202017087662
(71) Applicant: Biosense Webster (Israel) Ltd, Yokneam, 2066717 (IL)
(72) Inventor: URMAN, Roy, Irwindale 91706 (US); MIZRAHI, Liron Shmuel, Yokneam 2066717 (IL)
(74) Representative: Carpmaels & Ransford LLP

(57) **Abstract**

A method includes presenting to a user a three-dimensional (3D) map of at least part of an organ of a patient, the 3D map generated by a position-tracking system. An artificial object, which is non-trackable by the position-tracking system, is identified in a medical image of at least part of the organ. A graphical representation of the non-trackable artificial object is presented to the user on the 3D map.

## Description

### FIELD OF THE INVENTION

The present invention relates generally to mapping of body organs, and particularly to identifying natural features and/or artificial elements in medical images and incorporating the identified natural features and/or artificial elements in a three-dimensional (3D) map of the organ.

### BACKGROUND OF THE INVENTION

Registering medical images, such as ultrasound images, with an electrophysiological (EP) map was proposed in the patent literature. For example, U.S. Patent No. 7,517,318 describes system and method for imaging a target in a patient's body that includes the steps of providing a pre-acquired image of the target and placing a catheter having a position sensor, an ultrasonic imaging sensor and at least one electrode, in the patient's body. Positional information of a portion of the catheter in the patient's body is determined using the position sensor and electrical activity data-points of a surface of the target are acquired using the at least one electrode. An ultrasonic image of the target is obtained using the ultrasonic imaging sensor and positional information for the electrical activity data-points of the surface of the target is determined. An electrophysiological map of the target is generated based on the electrical activity data-points and the positional information for the electrical activity data-points. Positional information for any pixel of the ultrasonic image of the target is determined and the pre-acquired image and the electrophysiological map are registered with the ultrasonic image. The registered pre-acquired image, the electrophysiological map and the ultrasonic image are displayed on a display.

Algorithms to identify intrabody artificial objects using medical images, such as ultrasound images, were proposed in the patent literature. For example, U.S. Patent No. 9,317,920 describes a computer-based system and method(s) to detect and identify implanted medical devices ("IMDs") and/or retained surgical foreign objects ("RSFOs") from diagnostic medical images. Software tools based on pattern/object recognition and computer vision algorithms (but not limited only to these) are disclosed that are capable of rapid recognition of IMDs on x-rays ("XRs"), computer tomography ("CT"), ultrasound ("US"), and magnetic resonance imaging ("MRI") images. In some embodiments, the system provides further identification-information on the particular IMD and/or RSFO that has been recognized. For example, the system could be configured to provide information feedback regarding the IMD, such as detailed manual information, safety alerts, recalls, assess its structural integrity, and/or suggested courses of action in a specific clinical setting/troubleshooting. Embodiments are contemplated in which the system is configured to report possible 3D locations of RSFOs in the surgical field/images.

### SUMMARY OF THE INVENTION

An embodiment of the present invention that is described hereinafter provides a method including presenting to a user a three-dimensional (3D) map of at least part of an organ of a patient, the 3D map generated by a position-tracking system. An artificial object, which is non-trackable by the position-tracking system, is identified in a medical image of at least part of the organ. A graphical representation of the non-trackable artificial object is presented to the user on the 3D map.

In some embodiments, presenting the non-trackable artificial object includes calculating a registration between respective coordinate systems of the 3D map and the medical image, and overlaying the graphical representation of the non-trackable artificial object on the 3D map using the registration.

In some embodiments, identifying the non-trackable artificial object includes identifying in the medical image a known geometrical property of the non-trackable artificial object.

In an embodiment, identifying the known geometrical property of the non-trackable artificial object includes identifying at least one of a dimension of the object, a distance between components of the object, and a distinctive shape of the object.

In another embodiment, the distinctive shape of the non-trackable artificial object includes one of a circular shape and a tip shape.

In some embodiments, presenting the graphical representation includes presenting an artificial icon having an appearance of the non-trackable artificial object.

In an embodiment, the artificial icon includes a silhouette or an outline of the non-trackable artificial object.

In some embodiments, identifying the non-trackable artificial object includes identifying a position and orientation of the non-trackable artificial object in the medical image, and presenting the graphical representation on the 3D map with the same position and orientation.

In other embodiments, the 3D map includes a 3D electrophysiological (EP) map of at least a portion of a heart.

In some embodiments, the non-trackable artificial object includes one of a needle, a sheath, a tube, a surgical clamp, an artificial valve and a catheter.

There is additionally provided, in accordance with another embodiment of the present invention, a method including presenting to a user a three-dimensional (3D) map of at least part of a heart of a patient, the 3D map generated by a position-tracking system. A septum of the heart is identified in a medical image of at least part of the heart. A graphical representation of a location over the septum for transseptal puncture is presented to the user on the 3D map.

In some embodiments, presenting the location for transseptal puncture includes specifying the location using a machine learning algorithm.

There is further provided, in accordance with yet another embodiment of the present invention, a system including a display and a processor. The display is configured to present to a user a three-dimensional (3D) map of at least part of an organ of a patient, the 3D map generated by a position-tracking system. The processor is configured to identify an artificial object, which is non-trackable by the position-tracking system, in a medical image of at least part of the organ, and present to the user, on the 3D map, a graphical representation of the non-trackable artificial object.

There is furthermore provided, in accordance with another embodiment of the present invention, a system including a display and a processor. The display is configured to present to a user a three-dimensional (3D) map of at least part of a heart of a patient, the 3D map generated by a position-tracking system. The processor is configured to identify a septum of the heart in a medical image of at least part of the heart, and present to the user, on the 3D map, a graphical representation of a location over the septum for transseptal puncture.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a system for ultrasound (US) imaging and electrophysiological (EP) mapping a heart of a patient, in accordance with an embodiment of the present invention;
Fig. 2 is a flow chart that schematically illustrates a method for identifying non-navigated anatomical features and/or artificial elements in ultrasound images and representing the identified features and/or elements in a 3D electrophysiological (EP) map, in accordance with an embodiment of the present invention; and
Fig. 3 is a 3D electrophysiological (EP) map incorporating representations of non-navigated anatomical features and/or artificial elements identified in an ultrasound image, in accordance with an embodiment of the present invention.

### DETAILED DESCRIPTION OF EMBODIMENTS

### OVERVIEW

A three-dimensional (3D) map of an organ, such as a 3D electrophysiological (EP) map of a portion of a heart, can be generated using a mapping system (e.g., by a catheter-based electrical position-tracking system and/or magnetic position-tracking system).

In order to track an object in the heart by a position-tracking system, the object needs to have at least one position sensor of the position-tracking system coupled thereto. An object having such a sensor, and in some cases multiple sensors, is referred to herein as a "trackable object" or a "navigated object." Examples of such objects include electrodes and/or a distal end of a shaft and/or an expandable frame of a catheter.

When tracking a trackable object, the position-tracking system typically has information as to the position and orientation of the object, and can therefore present it on the 3D map (which was also generated by the position-tracking system) in any suitable way.

In practice, however, a medical procedure may involve other objects that do not have a position sensor of the position-tracking system. Such objects are referred to herein as "non-trackable objects" or "non-navigated objects." Examples of such objects include tubes, surgical clamps, needles, and catheter sheaths.

While not trackable by the position-tracking system, it is nevertheless important to visualize non-trackable objects on the 3D map during the medical procedure.

Embodiments of the present invention enable visualization of both trackable and non-trackable objects. The disclosed technique is based on identifying non-trackable objects in a medical image (e.g., ultrasound image) of an organ (e.g., heart) that is registered with the 3D map.

In some embodiments a processor presents to a user on a display, a 3D map of at least part of an organ (e.g., heart) of a patient, where the 3D map was generated, or is being generated, by a position-tracking system. The processor identifies an artificial object, which is non-trackable by the position-tracking system, in a medical image (e.g. US image) of at least part of the organ. Then, the processor presents to the user, on the 3D map, a graphical representation of the non-trackable artificial object.

In an embodiment, the processor first calculates a registration between respective coordinate systems of the 3D map and of the medical image, and then overlays the graphical representation of the non-trackable artificial object on the 3D map using the registration. One example of a graphical representation of the non-trackable artificial object is an artificial icon of the object overlaid on the 3D map.

Artificial elements (e.g. objects), such as the aforementioned tube, needle, surgical clamp, and balloon/basket/focal catheters and/or their sheaths, have well-defined geometrical properties and dimensions, as well as very distinct ultrasound reflections. Such artificial elements are therefore relatively simple to detect (or for parts of them to be detected) in the ultrasound image. Still, interpretation and proper representation of artificial elements is challenging. In some embodiments of the present invention, the processor uses a database of pre-known (e.g., prespecified) physical dimensions of the identified artificial element (e.g., length, French size, electrode positions, etc.). The processor may use this information both to identify the artificial element reliably in the medical image, and/or to create a realistic representation (e.g., silhouette) of the object on the 3D map.

Moreover, anatomical objects may be hard to detect and identify, as these tend to have complicated shapes, that are patient specific. Detecting, identifying and tagging clinically significant anatomical objects seen on the medical (e.g., US) image may well require a trained operator, including to manually tag, or put pointers, on the 3D map of such features. For example, during left atrial (LA) catheterization, it is often necessary to perform a transseptal puncture, i.e., to pierce the septum between the right atrium and the left atrium to gain access for the catheter into the LA. At present, a physician uses a medical image, such as an US image, to manually identify a transseptal point for piercing. The physician checks that the point is correct by pushing it with a needle or a sheath, observing the tenting produced in the US images, and uses the amount of tenting produced to confirm that this is the correct point. However, it would be useful to automate both the non-navigated needle or sheath (as described by the above embodiments) and the identified transseptal penetration point, and the subsequent representation process of such, in the 3D map.

Another disclosed technique, therefore, relates to automatic identification of a suitable/preferred location for transseptal puncture. In some embodiments, a processor uses machine-learning/image-processing algorithms to detect and identify non-navigated anatomical features in the image medical. The processor represents such features on the 3D map of the organ. In an embodiment, a processor applies a machine-learning (ML) algorithm to identify prespecified anatomical features in the acquired medical (e.g., US) image. For example, an ML algorithm is disclosed that is capable of identifying a transseptal penetration point of the septum in an US image. As transseptal puncture procedures are done frequently, the algorithm can be trained using a ground truth database of transseptal points that were manually identified and marked (e.g., tagged) by trained operators. The disclosed embodiments automatically identify the transseptal point and present it on the 3D EP map.

When a processor running the algorithm detects a non-navigated object, be it an artificial object or anatomical location, the processor generates an indication (e.g., an artificial icon, or a marker) of the objects' existence and position, at the time that the medical (e.g., US) image was acquired, to be incorporated into the 3D EP map. If, in a later image, if medical images are taken during a procedure, the non-navigated object is detected at a different position, the existing indication may be erased and a new indication incorporated into the 3D EP map.

The indication may, for example, be a marker to an anatomical object (e.g., feature), such as of a transseptal penetration point.

Typically, the processor is programmed in software containing a particular algorithm that enables the processor to conduct each of the processor related steps and functions outlined above.

By providing means to detect, model, and represent non-navigated anatomical features and/or artificial elements and to update (e.g., refresh) the representation on a 3D EP map, a physician may be more informed, in real time, while performing diagnostic and therapeutic EP procedures, so as to achieve a higher success rate in these typically complicated clinical procedures.

### SYSTEM DESCRIPTION

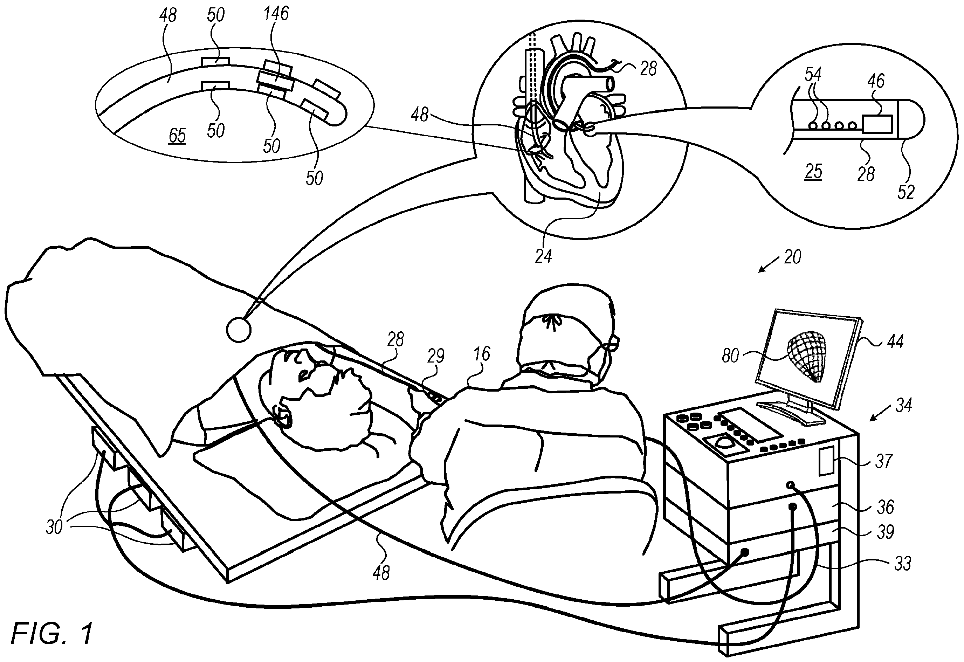

Fig. 1 is a schematic, pictorial illustration of a system 20 for ultrasound (US) imaging and electrophysiological (EP) mapping of a heart 24 of a patient, in accordance with an embodiment of the present invention. System 20 comprises a catheter 28, which is percutaneously inserted by a physician 16 into a chamber or vascular structure of the heart. Catheter 28 typically comprises a handle 29 for the physician to operate the catheter. Suitable controls on handle 29 enable physician 16 to steer, position and orient the distal end of the catheter as desired.

In the shown embodiment, as seen in inset 25, the distal end of a mapping and ablation catheter 28 includes a distally placed mapping/ablation electrode 52 to measure the electrical properties of the heart tissue. The distal end of the mapping catheter further includes an array of non-contact electrodes 54 to measure far field electrical signals in the heart chamber.

System 20 contains electronic circuitry to generate an electrical activation map, and can be used in conjunction with other mapping catheters, such as a multi electrode basket, Lasso^{™}, Pentray^{™} and balloon catheters.

Typically, mapping/ablation catheter 28 is introduced first, and a 3D EP map 80, generated from its data, is displayed on a monitor 44 and saved to a memory 37. Afterward, an ultrasound imaging catheter 48, shown in inset 65, is introduced. The two catheters may be introduced via the same or different vascular approaches. In the shown embodiment, catheter 28 and catheter 48 are both incorporated in system 20 and inserted concurrently into the heart via different vascular approaches. In this example, catheter 28 functions as an EP mapping catheter, and catheter 48, using an array of acoustic transducers 50, functions as an ultrasound imaging catheter.

System 20 enables physician 16 to perform a variety of mapping and imaging procedures, such as displaying real-time two-dimensional ultrasound images, and registering, overlaying or tagging structures in the patient's body, based on two-dimensional ultrasound images with 3D map 80.

To this end, system 20 comprises one or more positioning subsystems that measure three-dimensional location information and orientation coordinates of catheter 28. In one embodiment, the positioning subsystem comprises a magnetic position-tracking system comprising a set of external radiators 30, such as field-generating coils, which are located in fixed, known positions external to the patient. Radiators 30 generate fields, typically electromagnetic fields, in the vicinity of the heart 24.

A position sensor 46 inside catheter 28 (seen in inset 25) transmits, in response to the sensed fields, position-related electrical signals over cables 33 running through the catheter to a console 34. Console 34 comprises a processor 36 that calculates the location and orientation of catheter 28 based on the signals sent by a location sensor 46. Processor 36 typically receives, amplifies, filters, digitizes, and otherwise processes signals from catheter 28. Magnetic position tracking systems that may be used for this purpose are described, for example, in U.S. Pat. Application Publication Nos. 2004/0147920, and 2004/0068178, whose disclosures are incorporated herein by reference.

In another embodiment, the positioning subsystem comprises an electrical position-tracking subsystem, such as an Active Current Location (ACL) system, made by Biosense-Webster (Irvine California), which is described in US Patent No. 8,456,182, whose disclosure is incorporated herein by reference. In the ACL system, during an EP mapping procedure, the locations of electrodes 52 and/or 54 of catheter 28 are tracked while they are inside heart 24 of the patient. For that purpose, electrical signals are passed between electrodes 52 and/or electrodes 54 and body surface electrodes (not shown). Based on the signals, and given the known positions of the body surface electrodes on the patient's body, processor 36 calculates an estimated location of electrodes 52/54 within the patient's heart.

As noted above, system 20 employs a US catheter 48 in order to acquire ultrasound images which are analyzed by processor 36 to identify US-imaged non-navigated objects (e.g., anatomical features and/or artificial objects), including tissue locations and artificial elements. The US images of the heart may be produced during a cardiac EP mapping procedure (for example using CARTOSOUND^{®}), and typically shows navigated and non-navigated artificial objects within the heart in relation with anatomical features, such as wall tissue.

In some embodiments, the tip of US catheter 48 comprises a position sensor of the positioning subsystem, which is used for registration between the US image and EP map 80. Using position signals from the position sensor at the tip of US catheter 48 (e.g., a SOUNDSTAR^{®} catheter), the processor registers a coordinate system of the US image with that of 3D EP map 80, and presents representations of the non-navigated objects seen in the US image may be incorporated into the EP map. A catheter such as US catheter 48 is described in U.S. Patent Application Publication No. 2011/0152684, whose disclosure is incorporated herein by reference

Catheter 48 has acoustic transducers 50 that are adapted to emit sound waves and receive reflections from natural and artificial interfaces inside the heart. As seen, US catheter 48 has a magnetic location sensor 146 that is used to determine the position and orientation of US catheter 48 within the body. Using the position and orientation information, the reflections are then analyzed to construct both two-dimensional and three-dimensional images of the heart. System 20 comprises an ultrasound driver 39 that drives ultrasound transducers 50.

In an alternative embodiment, a hybrid catheter, which is capable of both ultrasound imaging functions and data acquisition (suitable for electrical activation map generation) can be used. Such catheters are described, for example, in U.S. Pat. Nos. 6,773,402, 6,788,967, and 6,645,145. Use of such catheters may permit the medical procedure to be shortened. In this alternative, only one catheter need be inserted. In all of the alternatives, as explained in further detail below, the electrical activation map is usually acquired first, and then used with the ultrasound images to assist in the interpretation of the latter. Suitable image registration techniques to coordinate the two modalities are disclosed in U.S. Pat. No. 6,650,927 and in co-pending application Ser. No. 11/215,435, both of common assignee herewith, and herein incorporated by reference.

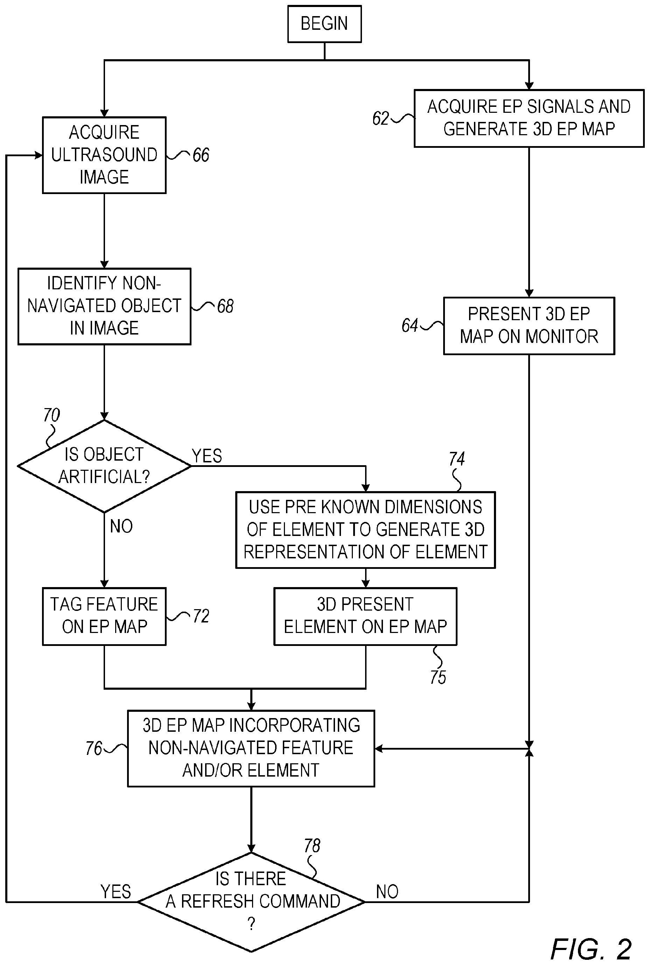

Processor 36 is typically programmed in software to carry out the functions described herein. The software may be downloaded to the processor in electronic form, over a network, for example, or it may, alternatively or additionally, be provided and/or stored on non-transitory tangible media, such as magnetic, optical, or electronic memory. In particular, processor 36 runs a dedicated algorithm as disclosed herein, such as included in Fig. 2, that enables processor 36 to perform the disclosed steps, as further described below.

### IDENTIFYING NON-NAVIGATED OBJECTS IN US IMAGE AND

### INCORPORATING THE OBJECTS INTO A 3D EP MAP

Fig. 2 is a flow chart that schematically illustrates a method for identifying non-navigated anatomical features and/or artificial elements in ultrasound images and representing the identified features and/or elements in a 3D electrophysiological (EP) map, in accordance with an embodiment of the present invention. The algorithm according to the presented embodiment carries out a process that begins with processor 36 generating (e.g., by processing EP data from catheter 28, or by uploading the EP map from memory 37) 3D EP map 80, at an EP map generation step 62. Next, processor 36 presents map 80 on monitor 44, at EP map displaying step 64.

At an ultrasound image acquisition step 66, processor 36 acquires an ultrasound image using catheter 48, as described in Fig. 1.

Using machine learning (ML) or image-processing techniques, processor 36 identifies a non-navigated anatomical feature (e.g., an LAA wall tissue transseptal location) and/or an artificial element (e.g., a needle) in the image, at a non-navigated feature and/or element identification step 68.

The processor checks, based on the data and predefined criteria, if the non-navigated anatomical features and/or artificial element is indeed a natural feature, or an artificial element, at an object type checking step 70.

If the object is natural, and is prespecified as a target for identification, the processor tags the identified (and already registered) location of the feature on the 3D EP map, at a natural feature tagging step 72.

If the object is deemed artificial, the processor, using a database of pre-known (e.g., prespecified) physical dimensions of the elements (e.g., length, French size, electrode positions, etc.) creates a realistic representation (e.g., silhouette) of the element, at a 3D element representation generation step 74. Then the processor incorporates the representation of step 74 on the 3D EP map, at an artificial element representation incorporation step 75.

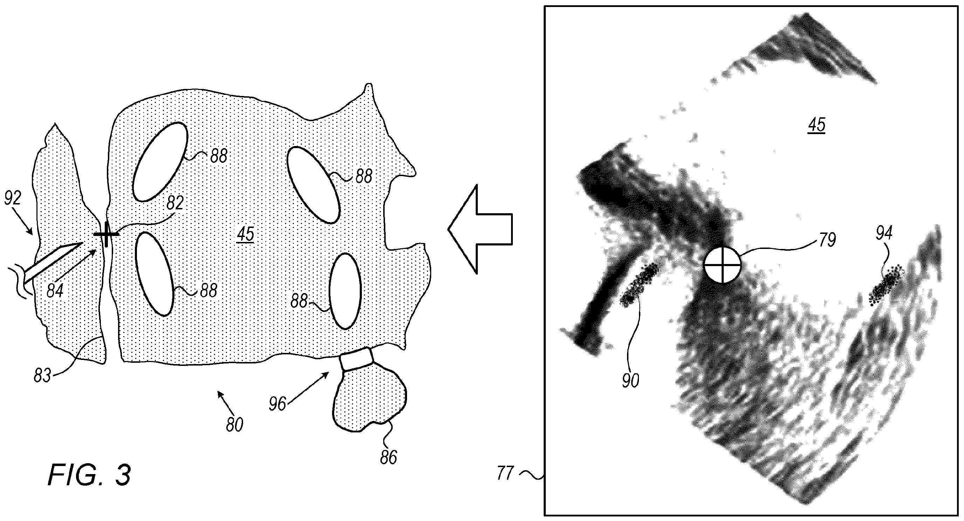

The result of steps 72-75 is an EP map incorporating non-navigated features and/or elements (step 76), such as EP map 80, as shown in detail in Fig. 3.

Finally, if the processor is requested to refresh the acquired US image (automatically or by a user) at a refreshing step 78, steps 66-75 are then repeated and the processor updates the 3D EP map accordingly. For example, if the processor detects a certain non-navigated element at a new position and/or orientation, the processor generates and presents an updated 3D representation of the element on the 3D EP map.

The example flow chart shown in Fig. 2 is chosen purely for the sake of conceptual clarity. Typically, more than one element is analyzed, but the process of considering multiple elements is omitted from the purposely highly simplified flow chart.

Fig. 3 is a 3D electrophysiological (EP) map 80 incorporating representations of non-navigated elements 79, 90 and 94 identified in an ultrasound image 77, in accordance with an embodiment of the present invention. EP map 80 shows a general structure of a left atrium 45 comprising ostia 88 of the pulmonary veins, a left atrium appendage 86, and septum 83.

In Fig. 3, US image 77 comprises a natural anatomical feature 79 and artificial elements 90 and 94. Anatomical feature 79 is a transseptal penetration point into left atrium 45, identified by an ML algorithm, as described above. Element 90 is identified by the disclosed technique as a needle (initially inserted by physician 16 into the right atrium) configured to pierce the septum at the location of anatomical feature 79, in order, for example, to subsequently guide the physician to advance a catheter via the pierced septum.

Lastly, object 94 is identified by the disclosed technique as a surgical clamp on the left atrium appendage to suppress clot generation.

As seen in Fig. 3, using the algorithm described in Fig. 2, the processor identifies location 79 in image 77 as location 82 on 3D EP map 80, and tags location 82 with a tag 84 overlaid at location 82 on septum representation 83.

With the artificial elements, the processor generates, as described above, a 3D representation 92 of the identified needle 90, and a 3D representation 96 of the identified clamp 94, and incorporates them into map 80. As the needle and the clamp are pre-known objects, the processor is able to represent these accurately on the map, for example by simulating geometrical shadows of the elements.

The example illustration shown in Fig. 3 is chosen purely for the sake of conceptual clarity. Fig. 3 shows only parts relevant to embodiments of the present invention. Other system elements, such as a catheter that may have been introduced, are omitted for simplicity.

Although the embodiments described herein mainly address cardiac applications, the methods and systems described herein can also be used in other applications, such as in representing non-navigated elements inside other organs having lumens (e.g., lungs) using medical images.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### Aspects of the invention

1. A method for identification and visualization of non-navigated objects in medical images, the method comprising:
   presenting to a user a three-dimensional (3D) map of at least part of an organ of a patient, the 3D map generated by a position-tracking system;
   identifying an artificial object, which is non-trackable by the position-tracking system, in a medical image of at least part of the organ; and
   presenting to the user, on the 3D map, a graphical representation of the non-trackable artificial object.
2. The method according to aspect 1, wherein presenting the non-trackable artificial object comprises calculating a registration between respective coordinate systems of the 3D map and the medical image, and overlaying the graphical representation of the non-trackable artificial object on the 3D map using the registration.
3. The method according to aspect 1, wherein identifying the non-trackable artificial object comprises identifying in the medical image a known geometrical property of the non-trackable artificial object.
4. The method according to aspect 3, wherein identifying the known geometrical property of the non-trackable artificial object comprises identifying at least one of a dimension of the object, a distance between components of the object, and a distinctive shape of the object.
5. The method according to aspect 4, wherein the distinctive shape of the non-trackable artificial object comprises one of a circular shape and a tip shape.
6. The method according to aspect 1, wherein presenting the graphical representation comprises presenting an artificial icon having an appearance of the non-trackable artificial object.
7. The method according to aspect 6, wherein the artificial icon comprises a silhouette or an outline of the non-trackable artificial object.
8. The method according to aspect 1, wherein identifying the non-trackable artificial object comprises identifying a position and orientation of the non-trackable artificial object in the medical image, and presenting the graphical representation on the 3D map with the same position and orientation.
9. The method according to aspect 1, wherein the 3D map comprises a 3D electrophysiological (EP) map of at least a portion of a heart.
10. The method according to aspect 1, wherein the non-trackable artificial object comprises one of a needle, a sheath, a tube, a surgical clamp, an artificial valve and a catheter.
11. A method for identification and visualization of non-navigated objects in medical images, the method comprising:
   presenting to a user a three-dimensional (3D) map of at least part of a heart of a patient, the 3D map generated by a position-tracking system;
   identifying a septum of the heart in a medical image of at least part of the heart; and
   presenting to the user, on the 3D map, a graphical representation of a location over the septum for transseptal puncture.
12. The method according to aspect 1, wherein presenting the location for transseptal puncture comprises specifying the location using a machine learning algorithm.

## Claims

1. A system for identification and visualization of non-navigated objects in medical images, the system comprising:
a display configured to present to a user a three-dimensional (3D) map of at least part of an organ of a patient, the 3D map generated by a position-tracking system; and
a processor, which is configured to:
identify an artificial object, which is non-trackable by the position-tracking system, in a medical image of at least part of the organ; and
present to the user, on the 3D map, a graphical representation of the non-trackable artificial object.

2. The system according to claim 1, wherein the processor is configured to present the non-trackable artificial object by calculating a registration between respective coordinate systems of the 3D map and the medical image, and overlaying the graphical representation of the non-trackable artificial object on the 3D map using the registration.

3. The system according to claim 1, wherein the processor is configured to identify the non-trackable artificial object by identifying in the medical image a known geometrical property of the non-trackable artificial object.

4. The system according to claim 3, wherein the processor is configured to identify the known geometrical property of the non-trackable artificial object by identifying at least one of a dimension of the object, a distance between components of the object, and a distinctive shape of the object.

5. The system according to claim 4, wherein the distinctive shape of the non-trackable artificial object comprises one of a circular shape and a tip shape.

6. The system according to claim 1, wherein the processor is configured to present the graphical representation by presenting an artificial icon having an appearance of the non-trackable artificial object.

7. The system according to claim 6, wherein the artificial icon comprises a silhouette or an outline of the non-trackable artificial object.

8. The system according to claim 1, wherein the processor is configured to identify a position and orientation of the non-trackable artificial object in the medical image, and to present the graphical representation on the 3D map with the same position and orientation.

9. The system according to claim 1, wherein the 3D map comprises a 3D electrophysiological (EP) map of at least a portion of a heart.

10. The system according to claim 1, wherein the non-trackable artificial object comprises one of a needle, a sheath, a tube, a surgical clamp, and artificial valve and a catheter.

11. A system, comprising:
a display configured to present to a user a three-dimensional (3D) map of at least part of a heart of a patient, the 3D map generated by a position-tracking system; and
a processor, which is configured to:
identify a septum of the heart in a medical image of at least part of the heart; and
present to the user, on the 3D map, a graphical representation of a location over the septum for transseptal puncture.

12. The system according to claim 11, wherein the processor is configured to specify the location using a machine learning algorithm.
